# EUROPÄISCHE PATENTSCHRIFT

(11) **EP 0 593 555 B1**
(45) Veröffentlichungstag und Bekanntmachung des Hinweises auf die Patenterteilung: **11.10.1995**
(21) Anmeldenummer: 92914014.3
(22) Anmeldetag: 08.07.1992
(51) Int. Cl.: A61F 2/06, A61F 2/04

(54) **TRACHEALSTENT**
TRACHEAL STENT
EXTENSEUR TRACHEEN

(30) Priorität: 11.07.1991 DE 4122923
(43) Veröffentlichungstag der Anmeldung: 27.04.1994
(73) Patentinhaber: WILLY RÜSCH AG, D-71385 Kernen (DE)
(72) Erfinder: FREITAG, Lutz, D-4300 Essen (DE); SINGVOGEL, Armin, D-7148 Remseck 2 (DE); SCHMITT, Klaus, D-7064 Remshalden-Grunbach (DE)
(74) Vertreter: KOHLER SCHMID + PARTNER
(86) Internationale Anmeldenummer: DE9200570
(87) Internationale Veröffentlichungsnummer: WO9300869

(56) Entgegenhaltungen:
- EP-A- 0 481 365
- DE-A- 4 032 759
- DE-U- 9 201 093
- FR-A- 2 617 721
- FR-A- 2 667 783
- US-A- 4 728 328
- US-A- 4 795 465
- US-A- 5 019 040

## Beschreibung

Die Erfindung geht aus von einem Trachealstent für verengte, erkrankte Tracheal- und/oder Bronchialsegmente, bestehend aus einem Kunststoffschaft gemäß dem Oberbegriff vom Anspruch 1.

Ein derartiger Trachealstent ist durch die US-Patentschrift 4,795,465 bekanntgeworden.

Der bekannte Trachealstent besteht aus einem Trachealschenkel, der sich an einem Ende in zwei Bronchialschenkel verzweigt. Sowohl der Trachealschenkel, wie auch die Bronchialschenkel sind aus einem rohrförmigen Kunststoff gefertigt, der über seine gesamte axiale Länge eine Steifigkeit für ein formstabiles Lumen aufweist.

Trachealstents werden in der Regel dann eingesetzt, wenn der kurative Einsatz einer künstlichen Trachealprothese nicht mehr möglich oder zu risikoreich ist. Moribunden Tumorpatienten kann über geeignete palliative Maßnahmen das Innenvolumen der Trachea zur Atmung offengehalten werden, in dem über ein Endoskop in die verengte Luftröhre Platzhalter beziehungsweise rohrartige Stents eingeführt werden. Die bislang dazu verfügbaren Stents zeigen jedoch erhebliche Nachteile, da sie weder von der äußeren Form, der geringen mechanischen Widerstandskraft der Stentwandung, noch im Hinblick auf den Schleimtransport der natürlichen Schleimclearance und dem Anwendungszweck gerecht werden.

Der Erfindung liegt deshalb die Aufgabe zugrunde, den bekannten Trachealstent dahingehend weiterzubilden, daß er endoskopisch problemlos applizierbar ist, dem Druck von Tumoren oder Narben ausreichend Widerstand entgegensetzen und sich schnellen Druckschwankungen beim Hustenstoß anpassen kann. Der Sitz des bekannten Trachealstents in der natürlichen Trachea ist dahingehend zu optimieren, daß er mit einem möglichst geringen Druck auf die Schleimhaut wirkt.

Diese Aufgabe wird erfindungsgemäß dadurch gelöst, daß der Kunststoffschaft Mittel aufweist, die in einer ersten Ausgestaltung den Kunststoffschaft im Außenumfang verkleinern und in einer zweiten Ausgestaltung den Kunststoffschaft über die gesamte axiale Länge aufweiten und im Kunststoffschaft längs einer Achse ein Lumen ausbilden, das im Querschnitt die Form einer natürlichen Trachea aufweist, wobei der Kunststoffschaft im ventrolateralen Bereich rigid und im dorsalen Bereich hochelastisch ausgebildet ist.

Der erfindungsgemäße Trachealstent hat damit den wesentlichen Vorteil, daß er komplexe paradoxe plastoelastische Eigenschaften aufweist. Er läßt sich über ein Rohr hindurch problemlos applizieren, er kann über besondere Mittel dem Druck von Tumoren standhalten, indem diese Mittel Widerlager bilden und weiterhin ist es möglich, daß sich der erfindungsgemäße Trachealstent Druckschwankungen während des Hustenstoßes anpassen kann.

Die Mittel sind beispielsweise am Kunststoffsschaft außen anliegende Metallspangen aus einem Memory-Metall, die im ventrolateren Bereich des Kunststoffschaftes diesen extrem stabilisieren beziehungsweise versteifen und im dorsalen Weiterhin ist aus der US-A-4,728,328 ein Trachealstent bekannt, der röhrenförmig ausgebildet und von einer Trägerspirale umgeben ist. Die Spirale ist beschichtet und um den röhrenförmigen Stent herumgewickelt.

Bereich den Kunststoffschaft hochelastisch ausgebildet lassen. Dies ist beispielhaft dadurch möglich, daß der Kunststoffschaft aus einem Silikonmaterial gefertigt ist. Einem Hauptproblem bei Langzeitanwendungen kann damit erfolgreich entgegengetreten werden, nämlich der Mukostase. Für einen Schleimtropfen ist die Schwerkraft proportional zur Gasflußgeschwindigkeit zum Quadrat. Wird einer der bekannten Stents zur Lumenerweiterung in eine Tumorstenose eingebracht, sinkt hier die Flußgeschwindigkeit lokal auf ein Minimum, weil die zu durchströmende Fläche zu groß wird, und das Sekret sammelt sich im Stent an. Mit dem erfindungsgemäßen Trachealstent lassen sich hohe Gasflußgeschwindigkeiten dadurch erzeugen, daß beim Hustenstoß die Querschnittsfläche im hochelastischen dorsalen Bereich verkleinert wird. Der erfindungsgemäße Trachealstent kann den Hustenstoß dynamisch fortpflanzen. Dynamische Eigenschaften der echten Luftröhre beziehungsweise der Bronchialschenkel werden bestmöglich imitiert. Der hochelastische dorsale Teil des Kunststoffschaftes verhält sich wie eine flexible Membran und die Sekretreinigung läßt sich damit wesentlich verbessern.

Der erfindungsgemäße Trachealstent ist über ein Endoskop in die Trachea einfach einführbar. Eine zu überbrückende Stenose wird nach Bedarf bougiert und anschließend wird der an sich zusammengelegte Kunststoffschaft in die Trachea eingeschoben. Anschließend werden die Mittel am Kunststoffschaft derart aktiviert, daß sich der Kunststoffschaft entfaltet und eine dauerhafte Form einnimmt, die gewährleistet, daß ein Lumen ausreichender Größe aufrechterhalten wird.

Werden als Mittel Metallspangen mit einem Memory-Effekt eingesetzt (Shape-Memory-Alloy), so hat dies den Vorteil, daß sie je nach Temperaturbehandlung eine vorgegebene Querschnittsform einnehmen können und in axialer Richtung gesehen auch unterschiedlich stark die natürliche Trachea weiten.

Die Metallspangen sind mit dem Kunststoff, einer Folie, die eine Wandstärke zwischen 0,4 mm und 1,5 mm aufweist, innig verbunden. Im aufgeweiteten Zustand des Kunststoffschaftes kann somit eine natürliche Trachea weitgehend nachempfunden werden. Bei dem erfindungsgemäßen Trachealstent bilden die Spangen und die Folie einen Materialverband, der dem bekannten Kunststoffschaft entspricht und ihn ersetzt. Die Spangen bilden dabei ein zuverlässiges und stabiles Widerlager für den die natürliche Trachea einengenden Tumor.

Die Metallspangen sind geöffnet im Querschnitt cranial hufeisenförmig ausgebildet und im zusammengeschobenen Zustand der Spangen können sich die Spangenenden überlappen bzw. sie stoßen aneinander. Werden die Metallspangen geweitet, so sind die erkennbaren freien Enden dieser Spangen voneinander beabstandet. Werden die Spangen durch thermischen Einfluß geweitet, so ist die Wärme über das Endoskop nach plaziertem zusammengelegtem Trachealstent einzuleiten. Das Weiten der Spangen kann auch über die Körperwärme erfolgen.

Die Spangen weisen über die Länge des Stents eine unterschiedliche Querschnittsform auf, so daß sie im aufgeweiteten Zustand dem natürlichen Trachealknorpel an der jeweiligen Stelle möglichst nahekommen.

Werden die Spangen geringfügig stärker aufgeweitet als die an dieser Stelle vorgegebene Lumengröße der natürlichen Trachea, so erhält der Stent einen unverrückbaren Sitz. Der Stent ist cranial längsoval und caudal gueroval, wobei mittig im Querschnitt nahezu ein Kreisquerschnitt erreicht wird . Dies gewährleistet einen optimalen Sitz mit möglichst geringem Druck auf die Schleimhaut. Eine ausreichende Kapillardurchblutung ist sichergestellt, eine wichtige Voraussetzung für eine ausreichende Flowdynamik und der notwendigen Mukusclearance.

Werden die Metallspangen am Außenumfang des Kunststoffschaftes angeordnet, so hat dies den Vorteil, daß die Innenoberfläche des Lumens des Kunststoffschaftes zusätzlich einfach zu hydrophilisieren ist. Über diese Maßnahme wird die Funktion der Flimmerhärchen nachempfunden.

Die Spangen können vollständig von der Folie, die mit den Kunststoffschaft bildet, ummantelt sein, damit auch Spangenmaterial einsetzbar ist, das keine besondere Gewebeverträglichkeit aufweist, aber besonders vorteilhaft Rückstellkräfte dauerhaft entwickelt, die notwendig sind, um die vorgegebenen Lumengröße aufrechtzuerhalten.

Als Spangen können auch aufzuschäumende Kunststoffmaterialien eingesetzt werden, die keine metallischen Komponenten aufweisen. Ist die Folie des Kunststoffschaftes thermostabil, so können die Spangen über Wärmeeinleitung mit einem kleinen Außendurchmesser zu Spangen mit einem großen Außendurchmesser vergrößert werden.

Die Spangen sind über die Folie, die mit den Spangen den erfindungsgemäßen Kunststoffschaft bildet, beabstandet miteinander verbunden. Deshalb ist es möglich, eine Normprothese beliebig zu kürzen und an den Bedarf anzupassen. Der erfindungsgemäße Stent kann für den jeweiligen Bedarf vom Arzt zurechtgeschnitten werden. Eine umfangreiche Lagerhaltung verschiedener Stentgrößen kann somit entfallen.

Ist der erfindungsgemäße Trachealstent mit Bronchialstümpfen versehen, so weist die Bifurkation zur Achse des zentralen Kunststoffschaftes zwei unterschiedliche Winkel auf. Damit kann der Stent bestmöglich und verspannungsfrei an den natürlichen Verlauf der Bronchialsegmente angepaßt werden. Bevorzugt wird deshalb der Trachealstent stets als Y-Stent gefertigt und der Arzt kann ihn nach Bedarf an beliebiger Stelle kürzen.

Ausgestaltungen der Erfindung lassen es zu, daß die Spangen nur im Trachealteil des Stents ausgebildet sind und nicht in den Bronchusschenkeln. Ferner können auch Spangen eingesetzt werden, deren freie Spangenenden im zusammengeschobenen Zustand aneinanderstoßen. Im aufgeweiteten Zustand des Kunststoffschaftes sind die freien Enden dieser Spangen dann mehr oder weniger weit voneinander beabstandet. Sind an den Bronchusschenkeln des Stents keine Spangen vorgesehen, so können diese Abschnitte aus einer formstabileren Kunststoffolie gefertigt sein als derjenigen Kunststoffolie, die für den Trachealabschnitt verwendet wird.

Weitere Vorteile ergeben sich aus der Beschreibung und der beigefügten Zeichnung. Ebenso können die vorstehend genannten und die noch weiter aufgeführten Merkmale erfindungsgemäß jeweils einzeln oder in beliebigen Kombinationen miteinander verwendet werden. Die erwähnten Ausführungsformen sind nicht als abschließende Aufzählung zu verstehen, sondern haben vielmehr beispielhaften Charakter.

Die Erfindung ist in der Zeichnung dargestellt und wird anhand von Ausführungsbeispielen näher erläutert. Es zeigen:
- Fig. 1: einen erfindungsgemäßen Trachealstent mit einem Trachealteil und beiden Bronchusschenkeln;
- Fig. 2a: einen Schnitt IIa-IIa der Fig. 1;
- Fig. 2b: einen Schnitt IIb-IIb der Fig. 1;
- Fig. 2c: einen Schnitt IIc-IIc der Fig. 1.

Die Gegenstände der Figuren sind teilweise stark schematisiert und zeigen die Gegenstände nicht maßstäblich. Ferner sind die Gegenstände teilweise stark vergrößert dargestellt, damit ihr Aufbau besser gezeigt werden kann.

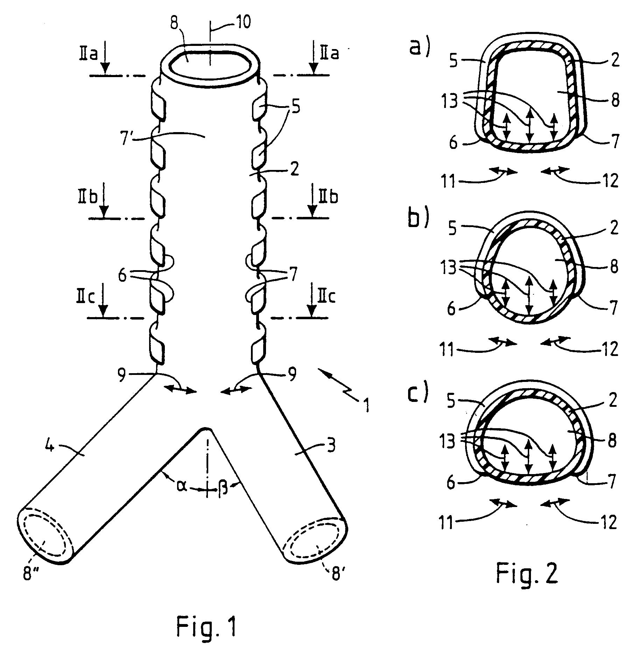

Fig. 1 zeigt einen aufgeweiteten Trachealstent 1 mit einem ersten Kunststoffschaft 2, einem zweiten Kunststoffschaft 3 und einem dritten Kunststoffschaft 4, wie er in einer natürlichen Trachea eingelegt sein kann und diese schient. Der erste Kunststoffschaft 2 entspricht dem Trachealanteil des Stents und der zweite, dritte Kunststoffschaft 3, 4 entspricht den Bronchusschenkeln.

In der Figur ist der erste Kunststoffschaft 2 von Spangen 5 umgriffen. Die Spangen 5 bilden einen innigen Materialverbund mit dem ersten Kunststoffschaft 2. Der erste Kunststoffschaft 2 ist aus einer Kunststofffolie gefertigt, die in hohem Maße gewebefreundlich ist. Die Materialstärke dieser Folie leigt zwischen 0,4 mm und 1,5 mm.

Die Spangen 5 sind in einer Ausführungsform aus Metall gefertigt, das in bekannter Weise Memory-Eigenschaften aufweist. In einer anderen Ausführungsform der Erfindung sind die Spangen 5 aus einem Kunststoffmaterial gefertigt, das in einem ersten Zustand flexibel und verformbar und in einem zweiten Zustand formstabil und porös ist.

Die Spangen 5 sind in der Fig. 1 mit freien Enden 6, 7 versehen, die voneinander beabstandet sind. Die Spangen 5 selbst sind in axialer Richtung des ersten Kunststoffschaftes 2 voneinander beabstandet und sind nur über das Kunststoffmaterial (Folie) des Kunststoffschaftes 2 miteinander verbunden. Die Spangen 5 geben dem ersten Kunststoffschaft 2 eine Formstabilität und halten im Kunststoffschaft 2 ein Lumen 8 offen. Die Spangen 5 umgreifen den ersten Kunststoffschaft 2 im aufgeweiteten Zustand nur ventrolateral und begrenzen mit den freien Enden 6, 7 einen Abschnitt 7' des Kunststoffschaftes 2 dorsal, der hochelastisch ausgebildet ist.

Die Spangen 5 können in Pfeilrichtungen 9 bewegt werden und zwar soweit, daß sich die freien Enden 6, 7 überlappen oder zumindest aneinanderstoßen. Werden die freien Enden 6, 7 aufeinander zugeführt, so kann der Kunststoffschaft 2 in einen kleineren Außenumfang überführt werden. Die Spangen 5 werden in der Anwendung so weit zusammengedrückt, daß sie in ein Lumen eines bekannten Endoskopes passen und durch dieses hindurchgeführt werden können.

Der zweite Kunststoffschaft 3 und der dritte Kunststoffschaft 4 sind im Ausführungsbeispiel der Fig. 1 aus einer nicht armierten Kunststofffolie gefertigt, die sofern sie aufgefaltet wird, ihre aufgefaltete Form beibehält, und ein Lumen 8', 8'' begrenzt. Der zweite und dritte Kunststoffschaft 3, 4 ist jeweils zu einer Achse 10 in einem Winkel α, der bevorzugt 36,3° beträgt und in einem Winkel β, der bevorzugt 46,3° beträgt, abgewinkelt. Die den Bronchialschenkeln entsprechenden Kunststoffschäfte 3, 4 lassen sich zusammendrücken, so daß sie durch die Stimmritze hindurch eingeführt werden können. Die Entfaltung der Kunststoffschäfte 3, 4 erfolgt unter Sicht durch den Trachealschenkel hindurch vorzugsweise mit Hilfe einer großen Faß- und Spreizzange.

Im Querschnitt kann der in der Figur 1 stark vereinfacht dargestellte Trachealstent längs der Achse 10 unterschiedliche Querschnittsformen aufweisen, die beispielhaft in den Figuren 2a, 2b, 2c dargestellt sind.

Fig. 2a zeigt cranial einen Schnitt in Pfeilrichtungen IIa-IIa der Figur 1. Der erste Kunststoffschaft 2 des Trachealstents ist von der Spange 5 ventrolateral umgeben, die formstabil die Folie des ersten Kunststoffschaftes 2 in einer Form hält, so daS das Lumen 8 aufrechterhalten wird. Der Stent weist in diesem Bereich eine längsovale Form auf. Die Spange 5 ist mit beabstandeten freien Enden 6, 7 gezeigt. Wird die Spange 5 in Pfeilrichtung 11, 12 zusammengedrückt, so kann der Außenumfang des Trachealstents in diesem Ausmaß verkleinert werden. Ist die Spange 5 bezüglich ihrer freien Enden 6, 7 übereinandergeschoben oder stoßen die freien Enden 6, 7 aneinander, so weist die Spange 5 entweder Rückstellkräfte auf, die es ermöglichen, daß sich die Spange 5 in eine Form selbsttätig ausweitet, wie sie Figur 2a zeigt, oder die Spange 5 ist beispielsweise über Wärmeeinfluß aktivierbar, so daß sie sich in die in der Figur 2a gezeigten Form entfaltet. In der Figur sind die freien Enden 6, 7 zur Spitze hin verjüngt ausgebildet.

Der hochelastische Abschnitt 7' kann schnelle Druckschwankungen aufnehmen und wellenartig fortpflanzen. In Pfeilrichtungen 13 kann dabei der Abschnitt 7' den Lumenquerschnitt pulsartig verkleinern und damit wird die Gasflußgeschwindigkeit sprunghaft erhöht.

Im Gegensatz zu Fig. 2a zeigen die Figuren 2b und 2c unterschiedliche Querschnittsformen der Spangen 5 im aufgeweiteten Zustand im mittigen bzw. caudalen Bereich. Über eine derartige Anordnung von Spangen 5 lassen sich unterschiedliche Lumenquerschnitte längs der Achse 10 (Fig. 1) einstellen.

Die Figur 1 und die Figur 2 zeigen den erfindungsgemäßen Trachealstent 1 im aufgeweiteten Zustand. In dem gezeigten Zustand hält er den Luftweg einer natürlichen Trachea zu den Bronchien hin offen.

Es versteht sich, daß auch der zweite und dritte Kunststoffschaft 3, 4 (Fig.1) von Spangen 5 ummantelt sein kann. Auch diese Spangen 5 können in der schon beschriebenen Form zusammengeschoben und wieder aufgeweitet werden.

## Patentansprüche

1. Trachealstent für verengte, erkrankte Tracheal- und Bronchialsegmente, bestehend aus einem
Kunststoffschaft (2, 3, 4), dadurch gekennzeichnet, daß der Kunststoffschaft (2) Mittel (5) aufweist, die in einer ersten Ausgestaltung den Kunststoffschaft (2) im Außenumfang verkleinern und in einer zweiten Ausgestaltung den Kunststoffschaft (2) über die gesamte axiale Länge aufweiten und im Kunststoffschaft (2) längs einer Achse (10) ein Lumen (8) ausbilden, das im Querschnitt die Form einer natürlichen Trachea aufweist, wobei der Kunststoffschaft (2) im ventrolateralen Bereich rigid und im dorsalen Bereich hochelastisch ausgebildet ist.

2. Trachealstent nach Anspruch 1, dadurch gekennzeichnet, daß die Mittel bewegbare Spangen (5) aus Metall oder Kunststoff sind, die am Außenumfang des Kunststoffschaftes (2) anliegen oder in den Kunststoffschaft (2) eingearbeitet sind.

3. Trachealstent nach Anspruch 1 oder 2, dadurch gekennzeichnet, daß die Innenoberfläche des Kunststoffschaftes (2) hydrophiliert ist.

4. Trachealstent nach einem der Ansprüche 1 - 3, dadurch gekennzeichnet, daß sich ein Ende des Kunststoffschaftes (2) zweischenklig verzweigt und weitere Kunststoffschäfte (3, 4) bildet.

## Claims

1. Tracheal stent for narrowed, diseased trachea and bronchial sections comprising a plastic shaft (2, 3, 4), characterized in that the plastic shaft (2) exhibits means (5) which, in a first manifestation, reduce the outer circumference of the plastic shaft (2) and, in a second manifestation, expand the plastic shaft (2) over the entire axial length to form a lumen (8) along an axis (10) of the plastic shaft (2), the lumen exhibiting a cross section of the shape of a natural trachea, whereby the plastic shaft (2) is rigid in the ventrolateral region and highly-elastic in the dorsal region.

2. Tracheal stent according to claim 1, characterized in that the means are movable clasps (5) made from metal or plastics, which seat in the outer circumference of the plastic shaft (2) or are worked into the plastic shaft (2).

3. Tracheal stent according to claim 1 or 2, characterized in that the inner surface of the plastic shaft (2) is hydrophilated.

4. Tracheal stent according to one of the claims 1 - 3, characterized in that one end of the plastic shaft (2) branches into two arms and forms additional plastic shafts (3, 4).

## Revendications

1. Extenseur trachéen pour segments trachéens et bronchiques pathologiques sténosés, composé d'une tige de matière plastique (2, 3, 4), caractérisé en ce que la tige de matière plastique (2) présente des moyens (5) qui, dans une première version, réduisent la circonférence externe de la tige de matière plastique (2) et dans une seconde version élargissent la tige de matière plastique (2) sur toute sa longueur axiale et forment dans la tige de matière plastique (2), le long d'un axe (10), une lumière (8) qui présente en section transversale la forme d'une trachée naturelle, la tige de matière plastique (2) étant rigide dans la partie ventrolatérale et très élastique dans la partie dorsale.

2. Extenseur trachéen selon la revendication 1, caractérisé en ce que les moyens sont des agrafes (5) mobiles en métal ou matière plastique qui sont accolées à la circonférence externe de la tige de matière plastique (2) ou sont incorporées dans la tige de matière plastique (2).

3. Extenseur trachéen selon la revendication 1 ou 2, caractérisé en ce que la surface interne de la tige de matière plastique (2) est rendue hydrophile.

4. Extenseur trachéen selon une des revendications 1 à 3, caractérisé en ce qu'une extrémité de la tige de matière plastique (2) se ramifie en deux branches et forme d'autres tiges de matière plastique (3, 4).
